# EUROPEAN PATENT APPLICATION

(11) **EP 1 884 260 A1**
(43) Date of publication of application: **06.02.2008**
(21) Application number: 07113108.0
(22) Date of filing: 25.07.2007
(51) Int. Cl.: A61M 39/22, A61M 39/26

(54) **Fluid mixture delivery instrument**

(30) Priority: 27.07.2006 JP 2006204771
(71) Applicant: Covidien AG, 8212 Neuhausen am Rheinfall (CH)
(72) Inventor: Kitani, Ichiro, Shizuoka-ken 437-0004 (JP); Sakai, Yosuke, Shizuoka-ken 437-0004 (JP); Fujiwara, Norifumi, Shizuoka-ken 437-0004 (JP)
(74) Representative: Körber, Martin Hans

(57) **Abstract**

To provide a fluid mixture delivery instrument with which it is possible to prevent the phenomenon of air remaining inside.
A mixture delivery instrument main body (10) of a fluid mixture delivery instrument (A) is configured by a chamber part (11), and a downstream branch pipe (12), an upper branch pipe (13) and an upstream branch pipe (14) which extend in different respective directions from the chamber part (11). Then, the inside of the chamber part (11) and a male luer part (28a) are linked in communication by the attachment of a rubber stopper (20) equipped with a slit (23) to the upper branch pipe (13), thereby closing off the upper branch pipe (13), and also by the insertion of the male luer part (28a) into the slit (23). Furthermore, a rubber stopper (20) is configured by a fixing piece (22) which is fixed to the aperture part side of the upper branch pipe (13), and a rubber stopper main body (21) which is joined to the fixing piece (22) and which is pushed inside the upper branch pipe (13) by means of the insertion of the male luer part (28a) into the slit (23). Then, when the rubber stopper main body (21) is pushed inside the upper branch pipe (13), the lower surface of the rubber stopper main body (21) becomes a roughly level surface.

## Description

### [Technical Field]

The present invention relates to a fluid mixture delivery instrument in which a plurality of infusion tubes or the like for medical purposes are joined and drug solutions or the like flow between all of these infusion tubes.

### [Technical Background]

Conventionally, certain drug solutions or physiological saline etc. are supplied inside the body of a patient using a plurality of infusion tubes, and in such cases all of the infusion tubes are linked in communication or blocked using a fluid mixture delivery instrument for a medical stopcock or the like. There are instruments such as this among fluid mixture delivery instruments which are equipped with a plurality of branch pipes and which have a rubber stopper which can be pierced by an injection needle or the like attached to the inside of certain of the branch pipes (see patent document 1, for example).
[Patent Document 1] Japanese Unexamined Patent Application Laid-Open 2004-16437

These fluid mixture delivery instruments are equipped with two branch pipes which extend horizontally from a main body portion and an injector connection port which is formed at the top of the main body portion. Then, provision is made in said injector connection part for a sealing valve body in which an insertion part which passes vertically through is formed. Consequently, it is possible for a twist-lock type injector and the inside of the main body of the fluid mixture delivery instrument to be linked in communication, by means of the insertion of the twist-lock type injector into the insertion part of the sealing valve body. By virtue of this, it is possible for a drug solution to be injected inside the main body of the fluid mixture delivery instrument from the twist-lock type injector, and for a drug solution to be extracted from the main body of the fluid mixture delivery instrument.

### [Disclosure of the Invention]

However, with a conventional fluid mixture delivery instrument as described above, when the twist-lock type injector is inserted into the insertion part of the sealing valve body, the lower part of the sealing valve body is pushed out into a space part inside the fluid mixture delivery instrument and protrudes. Consequently, there are problems in that air is liable to remain around the portion of the sealing valve body which is protruding into the space part inside the fluid mixture delivery instrument, and a troublesome operation is needed to remove the air inside the fluid mixture delivery instrument and inside the tubes of the infusion line. Furthermore, there are also problems in that bacteria are easily produced inside the fluid mixture delivery instrument due to the fact that air remains.

In view of this situation, the aim of the present invention is to provide a fluid mixture delivery instrument with which air is unlikely to remain inside.

In order to achieve the abovementioned aim, the features of the configuration of the fluid mixture delivery instrument pertaining to the present invention lie in the fact that it is equipped with a mixture delivery instrument main body comprising a chamber part and a plurality of branch pipes which extend in different respective directions from the chamber part and which include an upper branch pipe extending at least upwards; and a rubber stopper which is attached to the upper branch pipe and which closes off the upper branch pipe by means of the closure of a slit passing through the inside, and also which links in communication the inside of the chamber part and the inside of a connection pipe by means of the insertion of the connection pipe into the slit, and the fluid mixture delivery instrument is such that the rubber stopper is configured by a fixing piece which is fixed to the upper branch pipe and a rubber stopper main body which is joined to the fixing piece and which is pushed inside the upper branch pipe by means of the insertion of the connection pipe into the slit, and when the rubber stopper main body is pushed inside the upper branch pipe by means of the connection pipe, the lower surface of the rubber stopper main body which is positioned inside the upper branch pipe becomes a roughly level surface in a state in which it is in close contact with the inner peripheral surface of the upper branch pipe.

With the fluid mixture delivery instrument of the present invention configured in the manner described above, when a connection pipe, the male luer part of a syringe, for example, is inserted into the slit of the rubber stopper main body and the connection pipe links in communication with the chamber part, if the rubber stopper main body is pushed inside the upper branch pipe by means of the connection pipe, the lower surface of the rubber stopper main body which is positioned inside the upper branch pipe becomes a roughly level surface in a state in which it is in close contact with the inner peripheral surface of the upper branch pipe. Consequently, it is unlikely for a space part to be produced in which air can remain between the lower surface of the rubber stopper main body and the inner peripheral surface of the upper branch pipe, and the rubber stopper main body and the inner peripheral surface of the upper branch pipe.

In other words, no unevenness is produced on the lower surface of the rubber stopper main body, and therefore when a drug solution or the like flows inside the chamber part, the drug solution or the like goes through the whole portion of the space part inside the chamber part. Consequently, it is possible to suppress the phenomenon of air remaining inside the chamber part. As a result, it is no longer necessary to carry out the troublesome operation needed to remove the air inside the chamber part, and also it is possible to suppress the generation of bacteria inside the chamber part. Furthermore, when the supply of a drug solution or the like has finished and the connection pipe is taken out from the rubber stopper main body, the rubber stopper main body returns to its original position by means of the restoring force of the fixing piece, and the slit is closed off by means of the restoring force of the rubber stopper main body.

Moreover, the plurality of branch pipes pertaining to the present invention constitute pipes which are provided with fluid flow channels which respectively extend from the chamber part, and they are configured by the upper branch pipe and one other pipe or a plurality of other pipes. Furthermore, no provision is made for a valve body, or a stopcock type device which is provided with a valve body for linking prescribed branch pipes in communication, or for blocking them, and it is possible to use, as the fluid mixture delivery instrument, an instrument of the type in which drug solutions or the like are allowed to flow between branch pipes which are always linked in communication.

Furthermore, other features of the configuration of the fluid mixture delivery instrument pertaining to the present invention lie in the fact that the fixing piece of the rubber stopper is fixed to the aperture part side of the upper branch pipe. By virtue of this, when the supply of a drug solution or the like has finished and the connection pipe is taken out from the rubber stopper main body, the rubber stopper main body returns to a position on the aperture part side of the upper branch pipe by means of the restoring force of the fixing piece, and therefore the aperture part of the upper branch pipe can be reliably closed off.

Furthermore, other additional features of the configuration of the fluid mixture delivery instrument pertaining to the present invention lie in the fact that the fixing piece of the rubber stopper is configured by an upper fixing piece which is fixed to the aperture part side of the upper branch pipe, and a lower fixing piece which is fixed to the inside of the upper branch pipe. By virtue of this, when the rubber stopper main body is pushed inside the upper branch pipe, the upper fixing piece extends and the lower fixing piece contracts. Consequently, when the connection pipe is taken out from the rubber stopper main body, the rubber stopper main body returns to a position on the aperture part side of the upper branch pipe by means of the restoring force of both the upper fixing piece and the lower fixing piece, and the slit is closed off by means of the restoring force of the rubber stopper main body. In this case, the rubber stopper main body is supported by means of the upper fixing piece and the lower fixing piece, and therefore firm attachment is possible.

Furthermore, other additional features of the configuration of the fluid mixture delivery instrument pertaining to the present invention lie in the fact that the upper branch pipe forms a roughly cylindrical shape in which the diameter on the chamber part side portion is larger, and the diameter on the aperture side is smaller, and when the rubber stopper main body is pushed inside the upper branch pipe, it descends while deforming so as to expand in the horizontal direction.

When the connection pipe is inserted into the slit of the rubber stopper and the rubber stopper main body is pushed inside the upper branch pipe, the fixing piece or the portion around the joining part between the fixing piece and the rubber stopper main body extends, but it is possible for the movement distance of the rubber stopper main body to be made smaller due to the fact that the diameter of the chamber part side of the upper branch pipe, in other words the inside, is made larger. Consequently, the length by which the fixing piece or the portion around the joining part extends is also made smaller, and no excessive force is exerted on the fixing piece or the portion around the joining part. By virtue of this, the rubber stopper is unlikely to be damaged. Furthermore, the rubber stopper main body does not undergo any more than slight deformation, and also the lower surface of the rubber stopper main body can easily be made a level surface when it is pushed inside the upper branch pipe.

Furthermore, other additional features of the configuration of the fluid mixture delivery instrument pertaining to the present invention lie in the fact that the upper branch pipe is configured by a joining aperture edge part which is formed integrally with the chamber part and a roughly cylindrical separating member which is attached to the joining aperture edge part. By virtue of this, it is easy to produce a roughly cylindrical upper branch pipe with a large diameter in the chamber part side portion, and a small diameter on the aperture part side.

Furthermore, other additional features of the configuration of the fluid mixture delivery instrument pertaining to the present invention lie in the fact that a valve body which links in communication with an arbitrary branch pipe from among the abovementioned plurality of branch pipes by means of the movement of said valve body inside the chamber part is arranged inside the abovementioned chamber part. By virtue of this, it is possible to arbitrarily switch the state of linking in communication or blocking of each of the infusion tubes which are joined to the fluid mixture delivery instrument. Furthermore, the movement of said valve body involves rotation in the axial direction and movement in the axial direction.

### [Optimum Mode for Implementing the Invention]

### (First Mode of Implementation)

The fluid mixture delivery instrument pertaining to a first mode of implementation of the present invention will be described in detail below using the figures. Figure 1 to Figure 3 show the fluid mixture delivery instrument A pertaining to this mode of implementation, and said fluid mixture delivery instrument A is configured by a mixture delivery instrument main body 10, a rubber stopper 20 (see Figure 4 and Figure 5) attached inside the mixture delivery instrument main body 10, a cover member 25 and a valve body 30. Then, the mixture delivery instrument main body 10 is configured by a cylindrical chamber part 11 which is short in the axial direction and three branch pipes comprising a downstream branch pipe 12, an upper branch pipe 13, and an upstream branch pipe 14, which are joined maintaining an angle of 90 degrees to the outer peripheral surface of the chamber part 11.

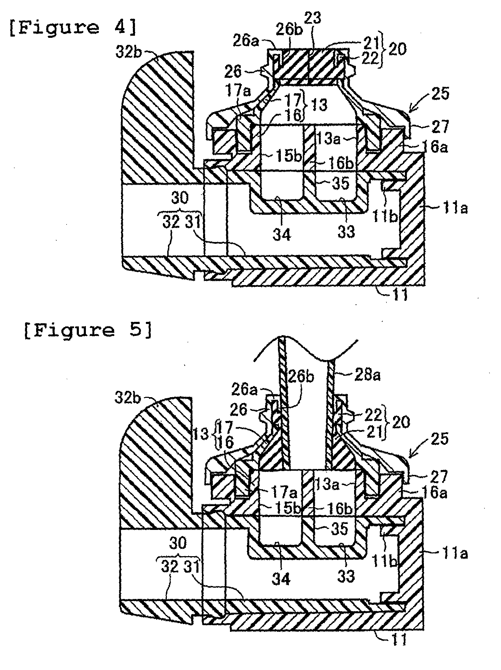

As shown in Figure 4 and Figure 5, the axial direction to the chamber part 11 is arranged facing to the front and rear directions (the left/right direction in Figure 4 and Figure 5), and a roughly cylindrical shape with a closed off rear part is formed. Then, a prescribed gap is provided on the inner surface of the rear wall part 11 a of the chamber part 11 between the inner peripheral surfaces of the chamber part 11, and an annular engagement part 11b which extends forwards is formed. Furthermore, through-holes 15a, 15b, 15c (see Figure 4 to Figure 6) are formed in roughly the centre of the axial direction of the chamber part 11. Among these through-holes 15a, 15b, 15c, the position of the centre part of the through-hole 15b is formed slightly offset to the front side than the position of the centre part of the other through-holes 15a, 15c.

The downstream branch pipe 12 is provided in a portion of the chamber part 11 corresponding to the through-hole 15a, and the inside of the chamber part 11 and a flow channel 12a which is formed inside the downstream branch pipe 12 are linked in communication by way of said through-hole 15a. Furthermore, the diameter of the through-hole 15b is set to be larger than the diameters of the through-holes 15a, 15c. A joining aperture edge part 16 which configures part of the upper branch pipe 13 is provided in a portion of the chamber part 11 corresponding to said through-hole 15b, and the inside of the chamber part 11 and the flow channel 13a which is formed inside the joining aperture edge part 16 are linked in communication by way of the through-hole 15b.

Furthermore, an auxiliary joining part 16a which surrounds the joining aperture edge part 16 in a state in which a gap is maintained with the joining aperture edge part 16 is formed on the outer peripheral side of the joining peripheral edge part 16, on the outer peripheral surface of the chamber part 11, and a partitioning wall 16b for partitioning the front and rear of the flow channel 13a is formed on both the left and right sides of the inner peripheral surface of the joining peripheral edge part 16, in a state of bridging. Furthermore, the upstream branch pipe 14 is provided in a portion of the chamber part 11 corresponding to the through-hole 15c, and the inside of the chamber part 11 and a flow channel 14a which is formed inside the upstream branch pipe 14 are linked in communication by way of said through-hole 15c.

The downstream branch pipe 12 is formed integrally with the chamber part 11, and it is configured by a base end part 12b on the chamber part 11 side, and a male luer part 12c on the tip end side which is formed to be narrower than the base end part 12b. Furthermore, the male luer part 12c is formed to taper at its end so that the tip end side portion is narrower than the base end part 12b side portion. Then, an engaging protruding part 12d is formed along the circumference of the outer peripheral surface of the downstream branch pipe 12, at the boundary part between the base end part 12b and the male luer part 12c.

The upper branch pipe 13 is configured by the joining aperture edge part 16 and the auxiliary joining part 16a which have been described above, and a roughly cap-shaped joining member 17. Said joining member 17 is configured by a short cylindrical body with a larger diameter on the lower part side, which gets steadily smaller moving upwards, and a lower end peripheral edge part 17a which is formed with a roughly annular shape is inserted between the joining aperture edge part 16 and the auxiliary joining part 16a, and fixed to the chamber part 11. Furthermore, the cover member 25 for fixing the rubber stopper 20 with the joining member 17 is attached to the outer peripheral side of the joining member 17.

The upper part 26 of the cover member 25 is formed with a short, roughly cylindrical shape with a size such that it can engage with the top outer periphery of the joining member 17, and the lower part 27 of said cover member is formed with a short, roughly cap shape with a larger diameter than that of the lower end peripheral edge part 17a of the joining member 17, and said shape is an ellipse which is long in the longitudinal direction when viewed in a plane. Then, the cover member 25 is detachably attached to the upper branch pipe 13 by means of the engagement of the upper part 26 with the top outer periphery of the joining member 17, and by means of the engagement of the lower end inner peripheral surface of the lower part 27 with the outer peripheral surface of the auxiliary joining part 16a. Furthermore, an annular ceiling part 26a extends from the upper end part towards the inside of the upper part 26 of the cover member 25, and at its tip end a roughly annular engagement piece 26b with a smaller diameter than the outer peripheral surface of the upper part 26 extends downwards. Then, the rubber stopper 20 is fixed by means of the engagement of said cover member 25 and the joining member 17.

The rubber stopper 20 is made of an elastic member such as synthetic rubber or an elastomer, and it is configured by a thick disc-shaped rubber stopper body 21 and a roughly cylindrical fixing piece 22 which is formed around the rubber stopper main body 21. The rubber stopper main body 21 and the fixing piece 22 are joined by each of their lower part side portions, and their upper part side portions are in a state of separation. Then, as shown in Figure 4, the rubber stopper 20 is attached to the cover member 25 by means of the insertion of the engagement piece 26b between the rubber stopper main body 21 and the fixing piece 22 in the rubber stopper 20, and the rubber stopper 20 is fixed by the joining member 17 and the cover member 25 by means of the attachment of the cover member 25 in this state to the upper branch pipe 13.

In other words, the upper part side portion of the fixing piece 22 is surrounded by the upper part 26, the ceiling part 26a and the engagement piece 26b of the cover member 25, and also the fixing piece 22 is prevented from breaking free from between the upper part 26 of the cover member 25 and the joining member 17, due to the fact that the joining part of the rubber stopper main body 21 and the fixing piece 22 in the rubber stopper 20 is pinched by the upper end part of the joining member 17 and the lower end part of the engagement piece 26b. By virtue of this, a state is achieved in which the fixing piece 22 is fixed and the rubber stopper main body 21 can move from the upper part 26 of the cover member 25 due to the fact that it is strongly push-pressured. Furthermore, when the rubber stopper main body 21 moves, the portion around the fixing piece 22 and the joining part between the fixing piece 22 and the rubber stopper main body 21 extends.

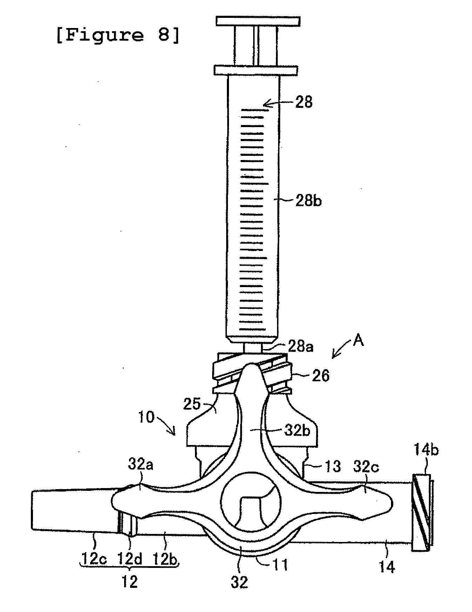

Furthermore, a slit 23 which passes through between the inside of the upper branch pipe 13 and the outside of the upper branch pipe 13 for forming part of the flow channel 13a of the upper branch pipe 13 is provided in the rubber stopper main body 21 of said rubber stopper 20. Said slit 23 is in a state of closure due to the elasticity of the rubber stopper 20 when the flow channel 13a of the upper branch pipe 13 is not in use. Furthermore, a flow channel can be formed which is linked in communication with the flow channel 13a inside a male luer part 28a by means of the insertion of the male lure part 28a of a syringe 28 (see Figure 8), for example, into the slit 23 of the rubber stopper 20 when the flow channel 13a of the upper branch pipe 13 is in use.

By virtue of this, a drug solution housing part 28b of the syringe 28 and the inside of the upper branch pipe 13 are linked in communication. Furthermore, at this time, the area between the male luer part 28a and the peripheral surface of the slit 23 reach a state of close contact due to the elasticity of the rubber stopper 20. In addition, the peripheral edge part of the lower surface of the rubber stopper main body 21 comes into close contact with the inner peripheral surface of the branch pipe 13. Then, the rubber stopper main body 21 is push-pressured downwards by means of the male luer part 28a, and its lower surface becomes a level surface, as shown in Figure 5. In other words, when said rubber stopper main body 21 is push-pressured downwards inside the upper branch pipe 13 by means of the male luer part 28a, and when the male luer part 28a is inserted inside the slit 23, the lower surface is preformed so as to become a level surface.

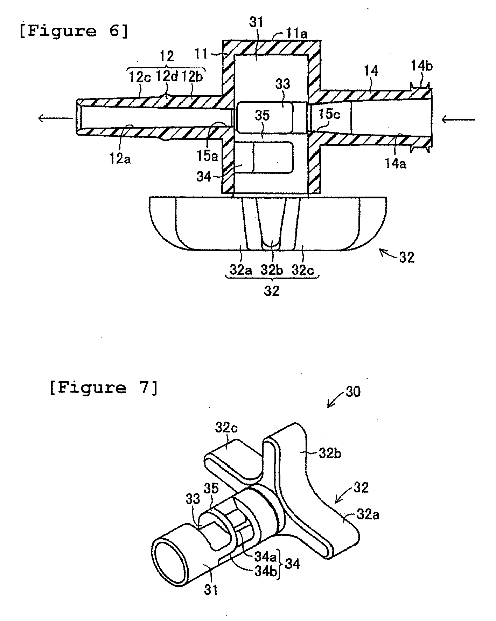

The upstream branch pipe 14 is integrally formed with the chamber part 11, and the flow channel 14a which comprises a tapered hole part is formed inside. Said flow channel 14a is linked in communication with the through-hole 15c, and a portion thereof on the through-hole 15c side is formed as a taper in which the diameter gets smaller closer to the through-hole 15c, and the diameter gets larger moving away from the through-hole 15c. Furthermore, the upstream side portion of the flow channel 14a (the right side portion in Figure 6) is formed as a taper in which the diameter gets steadily larger moving closer to the aperture part of the upstream branch pipe 14. Then, a joining screw part 14b is formed on the outer peripheral surface of the aperture part of the upstream branch pipe 14.

The valve body 30 is configured by a roughly cylindrical valve main body 31 and an operating part 32 which is joined to the front end part of the valve main body 31. Then, the valve main body 31 is arranged inside the chamber part 11, in a state in which its tip end part is inserted between the inner peripheral surface of the chamber part 11 and the engagement part 11b, and said valve main body rotates in the axial direction of the chamber part 11 by means of the operation of the operating part 32. Furthermore, as shown in Figure 7, two groove parts 33, 34 are formed in a row in the axial direction on the outer peripheral surface of the valve main body 31. The groove part 33 is configured by a cut-out groove which extends over a rough semi-circle along the circumference on a portion of the outer peripheral surface of the valve main body 31 which is slightly further rearwards than the centre in the axial direction.

Furthermore the groove part 34 is configured by a roughly L-shaped cut-out groove which comprises a circumferential direction groove part 34a which extends along the outer peripheral surface of the valve main body 31, parallel to the groove part 33 on a portion of the outer peripheral surface of the valve main body 31 which is slightly further forwards than the centre in the axial direction, and an axial direction groove part 34b which extends to the rear side in the axial direction, curving away from one end part of the circumferential direction groove part 34a. Then, the axial direction groove part 34b of the groove part 34 is provided in a position which maintains a prescribed gap with one end part of the groove part 33, and the other end part of the circumferential direction groove part 34a of the groove part 34 is positioned on one side (the front side in Figure 7) further along in the circumferential direction than the other end part of the groove part 33.

The length of the groove part 33 and the groove part 34 along the circumferential direction of the valve main body 31 is set to be both equal and roughly semicircular on the circumference, and the gap between the groove part 33 and the circumferential direction groove part 34a of the groove part 34, and the gap between the groove part 33 and the axial direction groove part 34b of the groove part 34 is set to be equal. Then, a barrier part 35 which runs along the outer peripheral surface of the valve main body 31 is formed between the groove part 33 and the circumferential direction groove part 34a of the groove part 34. Furthermore, the valve body 31 which is arranged inside the chamber part 11 is in a state in which the portion where the groove part 33 and the axial direction groove part 34b of the groove part 34 are formed matches the position of the through-holes 15a, 15c, and the portion where the circumferential direction groove part 34a of the groove part 34 is formed is opposite the front part side portion on the inner peripheral surface of the chamber part 11.

Furthermore, the portion where a barrier part 35 on the outer peripheral surface of the valve main body 31 is formed is in a state in which it faces a portion which is slightly further forwards than the through-hole 15b and the centre of the inner peripheral surface of the chamber part 11. Consequently, as shown in Figure 6, when the valve body 31 is positioned so that the barrier part 35 faces upwards, the groove part 33 is opposite the through-hole 15c, and the inside of the chamber part 11 and the upstream branch pipe 14 are linked in communication via the groove part 33. Furthermore, the rear part side of the axial direction groove part 34b in the groove part 34 is opposite the through-hole 15a, and the inside of the chamber part 11 and the downstream branch pipe 12 are linked in communication via the groove part 34.

In this case, the partitioning wall 16b is positioned over the barrier part 35, and the upper surface of the barrier part 35 and the lower surface of the partitioning wall 16b are in contact in a very close state. Then, a space part which forms the flow channel 13a of the upper branch pipe 13 is positioned on top of the partitioning wall 16b, and therefore the groove part 33 and the groove part 34 are linked in communication via the flow channel 13a. Accordingly, in this state, it is possible for a drug solution or the like to flow into the downstream branch pipe 12 from the upstream branch pipe 14, via the chamber part 11 and the upper branch pipe 13. In this case, the drug solution or the like which flows inside the groove part 33 from the upstream branch pipe 14 goes over the partitioning wall 16b and flows into the groove part 34. Consequently, the drug solution or the like passes through inside the flow channel 13a at the top of the chamber part 11, and it is possible to suppress the phenomenon of air or the like remaining inside the chamber part 11 and inside the flow channel 13a.

From this state, when the valve body 30 is rotated one way to make the groove part 33 face the through-hole 15a, and also the outer peripheral surface of the valve main body 31 is made to face the through-hole 15c, the inside of the chamber part 11 and the downstream branch pipe 12 are linked in communication, and there is a block between the inside of the chamber part 11 and the upstream branch pipe 14. Furthermore, from the state in Figure 6, when the valve body 30 is rotated the other way to maintain a state in which the groove part 33 is made to face the through-hole 15c, and also the outer peripheral surface of the valve main body 31 is made to face the through-hole 15a, there is a block between the inside of the chamber part 11 and the downstream branch pipe 12, and the inside of the chamber part 11 and the upstream branch pipe 14 are linked in communication.

In this way, it is possible for both the downstream branch pipe 12 and the upstream branch pipe 14 to be linked in communication with the inside of the chamber part 11, and for only one of them to be linked in communication with the chamber part 11, by means of the rotational operation of the valve body 30. Moreover, the operating part 32 is equipped with three operating pieces 32a, 32b, 32c, and said operating pieces 32a, 32b, 32c are formed maintaining an angle of 90 degrees, so as to correspond to the downstream branch pipe 12, the upper branch pipe 13 and the upstream branch pipe 14, respectively.

Furthermore, as shown in Figure 8, it is possible to detachably attach the syringe 28 to the upper branch pipe 13. Said syringe 28 is equipped with the drug solution housing part 28b which houses a drug solution or the like and the male luer part 28a which has a narrow diameter and is cylindrical in shape, and it is possible for the male luer part 28a to link the inside of the drug solution housing part 28b and the flow channel 13a of the upper branch pipe 13 in communication, by means of the insertion of the male luer part 28a inside the slit 23 of the rubber stopper 20. When said male luer part 28a is inserted inside the slit 23 of the rubber stopper 20, the male luer part 28a presses against the rubber stopper main body 21 at the inner peripheral surface of the joining member 17, and while moving it opens out the slit 23.

At this time, the joining part of the rubber stopper main body 21 and the fixing piece 22 turns around from upwards to downwards and extends. By virtue of this, the rubber stopper main body 21 is held down at the joining member 17 by means of the push-pressure force of the male luer part 28a, and it descends to a state in which it is in close contact with the inner peripheral surface of the joining member 17. Then, when the male luer part 28a is inserted inside the slit 23 and is linked in communication with the inside of the chamber part 11, as shown in Figure 5, the lower surface of the rubber stopper main body 21 becomes a level surface. Furthermore, there is close contact between the inner peripheral surface of the joining member 17 and the rubber stopper main body 21, and between the male luer part 28a and the peripheral surface of the slit 23 due to the elastic force of the rubber stopper 20.

Consequently, as shown in Figure 6, the upstream branch pipe 14 and the downstream branch pipe 12 are caused to be linked in communication, and while the drug solution or the like flows from the upstream branch pipe 14 side towards the downstream branch pipe 12 side, other drug solutions or the like from the syringe 28 can be mixed with said drug solution or the like, as shown in Figure 8. Furthermore, in a state in which there is a block between the inside of the chamber part 11 and the upstream branch pipe 14, other drug solutions or the like can flow from the syringe 28 to the downstream branch pipe 12.

With this configuration, in the case where two types of drug solution are supplied inside the body of the patient (not shown in the figures), the rear end part of an infusion tube (not shown in the figures) to which is connected an indwelling needle which is made to pierce the patient and remain there is firstly connected to the downstream branch pipe 12. Next, the male luer part provided at the tip end part of the infusion tube which extends from a container or the like housing one of the drug solutions to be supplied to the patient is connected to the upstream branch pipe 14. Next, in a state in which another drug solution is sucked inside the drug solution housing part 28b of the syringe 28, the male luer part 28a is made to penetrate the slit 23 of the rubber stopper 20.

Then, the drug solution passes inside the infusion line which includes the chamber part 11, and after all the air inside the infusion line has been expelled to the outside, and in a state in which the indwelling needle is made to pierce the patient's body and remain there, the drug solution is supplied to the patient by sending the drug solution in the container or the like to the patient. Furthermore, the drug solution inside the drug solution housing part 28b of the syringe 28 is also appropriately injected inside the chamber part 11 via the flow channel 13a of the upper branch pipe 13. When the air inside the infusion line in this case is expelled to the outside, the lower surface of the rubber stopper main body 21 becomes a level surface, and the inner peripheral surface of the joining member 17 and the rubber stopper main body 21, and the male luer part 28a and the peripheral surface of the slit 23 respectively reach a state of close contact.

Consequently, locations where air remains are unlikely to be created inside the chamber part 11. In other words, air is unlikely to remain in any of the space parts inside the chamber part 11 because these have become the flow channel for the drug solution. By virtue of this, the phenomenon of air being mixed in with the drug solution which is supplied to the patient is suppressed. Then, when the supply of drug solution from the syringe 28 is finished, and the male luer part 28a is withdrawn from the slit 23, the push-pressuring from the male luer part 28a on the rubber stopper main body 21 is released, and also the state shown in Figure 4 is returned to due to the restoring force of the fixing piece 22. Furthermore, thanks to this fluid mixture delivery instrument A the inside of the upper branch pipe 13 is closed off by the rubber stopper 20, and therefore it is possible to suppress the phenomenon of air entering the chamber part 11, and bacteria propagating.

In this way, with the fluid mixture delivery instrument A pertaining to this mode of implementation, when the male luer part 28a is inserted into the slit 23 and the syringe 28 is linked in communication with the chamber part 11, the lower surface of the rubber stopper main body 21 which is pushed inside the joining member 17 becomes a roughly level surface in a state in which it is in close contact with the inner peripheral surface of the joining member 17. Consequently, there are no longer any space parts where air might remain between the lower surface of the rubber stopper main body 21, the inner peripheral surface of the joining member 17 and the rubber stopper main body 21, and between male luer part 28a and the peripheral surface of the slit 23. As a result, it is possible to suppress the need for the troublesome operation for removing air inside the chamber part, and to suppress the generation of bacteria inside the chamber part.

Furthermore, with the fluid mixture delivery instrument A, when the rubber stopper main body 21 moves vertically, the joining member 17 which is in contact therewith forms a roughly cylindrical shape in which the lower part side portion diameter is larger and the upper part side portion diameter is smaller, and therefore when the rubber stopper main body 21 is pushed onto the lower part side of the joining member 17, it descends while deforming so as to expand in the horizontal direction. Consequently, the movement distance of the rubber stopper main body 21 becomes smaller, and the length of extension of the joining part of the rubber stopper main body 21 and the fixing piece 22 also becomes smaller. As a result, the fixing piece 22 etc. is not subjected to any excessive force, and it is possible to prevent damage to the rubber stopper 20. Furthermore, the amount of deformation of the rubber stopper main body 21 is also small, and also the lower surface of the rubber stopper main body 21 readily becomes a level surface when it descends. In addition, the upper branch pipe 13 is configured by the joining aperture edge part 16 which is integrally formed with the chamber part 11 and the joining member 17 which is attached to the joining aperture edge part 16, and therefore the upper branch pipe is easily produced.

### (Second Mode of Implementation)

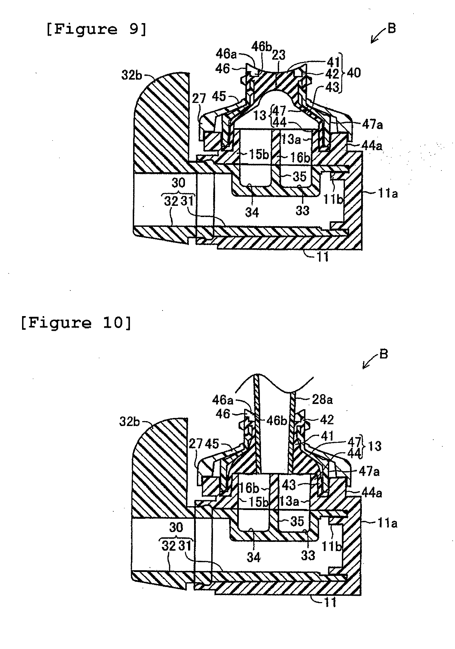

Figure 9 and Figure 10 show a fluid mixture delivery instrument B pertaining to a second mode of implementation of the present invention. With this fluid mixture delivery instrument B, the thickness of a lower end peripheral edge part 47a which is formed with a roughly annular shape on a joining member 47 is set to be less than the thickness of the lower end peripheral edge part 17a of the joining member 17. Furthermore, an annular ceiling part 46a curves and extends obliquely downwards and inwards from the upper end part of the upper part 46 of a cover member 45, and an engagement piece 46b whose thickness is set to be greater than that of the abovementioned engagement piece 26b extends downwards at its tip end. The upper surface of the rubber stopper main body 41 in the rubber stopper 40 is formed as a curved surface with the central part sinking downwards, and the lower surface of the rubber stopper 40 is formed as a curved surface in which the central part curves upwards with greater curvature than the upper surface.

Consequently, the rubber stopper main body 41 is formed roughly disc-shaped with a thick peripheral edge part and a thin central side, an upper fixing piece 42 is formed in the upper part side portion on the outer peripheral surface of the rubber stopper main body 41, and a long lower fixing piece 43 is formed to extend downwards to the lower end outer peripheral part of the rubber stopper main body 41. Then, the lower fixing piece 43 of the rubber stopper 40 is fixed, along with a lower end peripheral edge part 47a of a joining member 47, by insertion between a joining aperture edge part 44 and an auxiliary joining part 44a. Furthermore, the upper fixing piece 42 of the rubber stopper 40 is fixed by an engagement piece 46b of the cover member 45 and an upper end part of the joining member 47. The configuration of the other parts of this fluid mixture delivery instrument B is the same as that of the fluid mixture delivery instrument A which has been described above. Accordingly, a description of identical components having identical references will be omitted.

Because this configuration is adopted, when the male luer part 28a is inserted inside the slit 23 of the rubber stopper 40, the male luer part 28a presses against the rubber stopper main body 41 on the inside of the joining member 47, and while moving it opens out the slit 23. At this time, the rubber stopper main body 41 moves downwards, while the upper fixing piece 42 is made to extend, and also the lower fixing piece 43 is made to contract. Then, when the male luer part 28a is inserted inside the slit 23 and it is linked in communication with the inside of the chamber part 11, the lower surface of the rubber stopper main body 41 becomes a level surface, as shown in Figure 10.

In other words, this is because when said rubber stopper main body 41 is also push-pressured downwards inside the upper branch pipe 13 by means of the male luer part 28a and when the male luer part 28a is inserted inside the slit 23, the lower surface is preformed to become a level surface. Furthermore, a state of close contact due to the elasticity of the rubber stopper 40 is reached between the upper end part of the joining member 47 and the rubber stopper main body 41, and the male luer part 28a and the peripheral surface of the slit 23. According to this fluid mixture delivery instrument B, a space part in which air might remain is unlikely to be produced on the lower surface of the rubber stopper main body 41, and therefore it is possible to suppress the need for the troublesome operation for removing air inside the chamber part 11, and to suppress the generation of bacteria inside the chamber part.

Furthermore, when the male luer part 28a is removed from the rubber stopper main body 41, the rubber stopper main body 41 is returned to a position on the aperture part side of the joining member 47 due to the restoring force of both the upper fixing piece 42 and the lower fixing piece 43, and the slit 23 is closed off due to the restoring force of the rubber stopper main body 41. In this case, the rubber stopper main body 41 is supported by means of the upper fixing piece 42 and the lower fixing piece 43, and therefore it is firmly supported. Other efficacious actions of this fluid mixture delivery instrument B are the same as those of the fluid mixture delivery instrument A described above.

Furthermore, the fluid mixture delivery instrument pertaining to the present invention is not limited to the modes of implementation described above, but can be appropriately modified. For example, in the modes of implementation described above, a stopcock type device equipped with a valve body 30 is used as the fluid mixture delivery instrument, but a device may be used as the fluid mixture delivery instrument pertaining to the present invention which is not equipped with a valve body, but which allows the flow of drug solution or the like from the upstream branch pipe to the downstream pipe which is constantly linked in communication, and also which makes it possible for other drug solutions or the like to flow from the upper branch pipe to the chamber part. Furthermore, it is possible to use a fluid mixture delivery instrument which is not equipped with an upstream branch pipe, in which the branch pipes are configured by an upper branch pipe and a downstream branch pipe alone.

Furthermore, in the first mode of implementation described above, the fixing piece 22 of the rubber stopper 20 is formed with a roughly cylindrical shape which is formed around the rubber stopper main body 21, but said fixing piece 22 may be configured by two or more linear or band-shaped members which are arranged to maintain a gap on the peripheral surface of the rubber stopper main body. Similarly, the upper fixing piece 42 and the lower fixing piece 43 in the second mode of implementation may also be configured by a plurality of linear or band-shaped members.

### [Brief Description of the Figures]

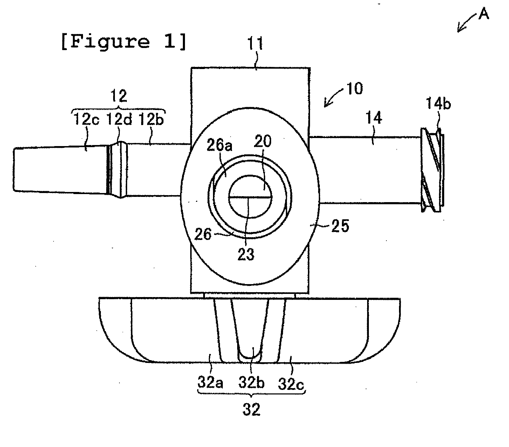

[Figure 1] is a plan view showing the fluid mixture delivery instrument pertaining to the first mode of implementation of the present invention.

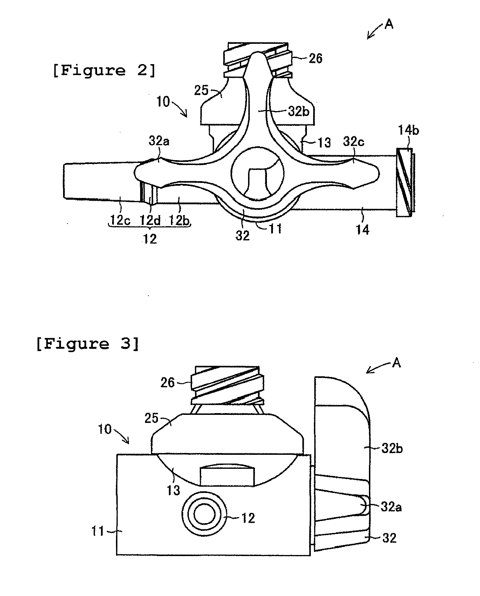

[Figure 2] is a front view of the fluid mixture delivery instrument.
[Figure 3] is a side view of the fluid mixture delivery instrument.
[Figure 4] is a cross-sectional view of the fluid mixture delivery instrument.
[Figure 5] is a cross-sectional view showing a state in which the male luer part is inserted into the rubber stopper of the fluid mixture delivery instrument of Figure 4.
[Figure 6] is a cross-sectional view showing the relationship between the main body and the valve body of the fluid mixture delivery instrument.
[Figure 7] is an oblique view showing the valve body.
[Figure 8] is a front view showing a state in which the syringe is attached to the fluid mixture delivery instrument.
[Figure 9] is a cross-sectional view of the fluid mixture delivery instrument pertaining to the second mode of implementation of the present invention.
[Figure 10] is a cross-sectional view showing a state in which the male luer part is inserted into the rubber stopper of the fluid mixture delivery instrument of Figure 9.

### [Explanation of Symbols]

10...mixture delivery instrument main body; 11...chamber part; 12...downstream branch pipe; 13...upper branch pipe; 14...upstream branch pipe; 16, 44...joining aperture edge part; 16a, 44a...auxiliary joining part; 17, 47...joining member; 20, 40...rubber stopper; 21, 41...rubber stopper main body; 22...fixing piece; 23...slit; 28a...male luer part; 30...valve body; 42...upper fixing piece; 43...lower fixing piece; A, B...fluid mixture delivery instrument.

## Claims

1. Fluid mixture delivery instrument equipped with a mixture delivery instrument main body comprising a chamber part and a plurality of branch pipes which extend in different respective directions from the abovementioned chamber part and which include an upper branch pipe extending at least upwards; and a rubber stopper which is attached to the abovementioned upper branch pipe and which closes off the abovementioned upper branch pipe by means of the closure of a slit passing through the inside, and also which links in communication the inside of the abovementioned chamber part and the inside of a connection pipe by means of the insertion of the abovementioned connection pipe into the abovementioned slit, which fluid mixture delivery instrument is **characterized in that** the abovementioned rubber stopper is configured by a fixing piece which is fixed to the abovementioned upper branch pipe and a rubber stopper main body which is joined to the abovementioned fixing piece and which is pushed inside the abovementioned upper branch pipe by means of the insertion of the abovementioned connection pipe into the abovementioned slit, and when the abovementioned rubber stopper main body is pushed inside the abovementioned upper branch pipe by means of the abovementioned connection pipe, the lower surface of the abovementioned rubber stopper main body which is positioned inside the abovementioned upper branch pipe becomes a roughly level surface in a state in which it is in close contact with the inner peripheral surface of the abovementioned upper branch pipe.

2. Fluid mixture delivery instrument according to Claim 1 in which the fixing piece of the abovementioned rubber stopper is fixed to an aperture part side of the abovementioned upper branch pipe.

3. Fluid mixture delivery instrument according to Claim 1 in which the fixing piece of the abovementioned rubber stopper is configured by an upper fixing piece which is fixed to the aperture part side of the abovementioned upper branch pipe, and a lower fixing piece which is fixed to the inside of the abovementioned upper branch pipe.

4. Fluid mixture delivery instrument according to any one of Claims 1 to 3, in which the abovementioned upper branch pipe forms a roughly cylindrical shape in which the diameter on the abovementioned chamber part side portion is larger, and the diameter on the abovementioned aperture side is smaller, and when the abovementioned rubber stopper main body is pushed inside the abovementioned upper branch pipe, it descends while deforming so as to expand in the horizontal direction.

5. Fluid mixture delivery instrument according to any one of Claims 1 to 4, in which the abovementioned upper branch pipe is configured by a joining aperture edge part which is formed integrally with the abovementioned chamber part and a roughly cylindrical separating member which is attached to the abovementioned joining aperture edge part.

6. Fluid mixture delivery instrument according to any one of Claims 1 to 5 in which a valve body which links in communication with an arbitrary branch pipe from among the abovementioned plurality of branch pipes by means of the movement of said valve body inside the abovementioned chamber part is arranged inside the abovementioned chamber part.
